(19) Europäisches Patentamt
European Patent Office
Office européen des brevets

(11) Numéro de publication : **0 145 618**
**B1**

(12) **FASCICULE DE BREVET EUROPÉEN**

(45) Date de publication du fascicule du brevet :
03.12.86

(51) Int. Cl.⁴ : **C 07 C102/00**

(21) Numéro de dépôt : **84420186.3**

(22) Date de dépôt : **30.10.84**

(54) **Procédé de préparation de la N,N'-bis(hydroxy-2 éthyl)oxamide.**

(30) Priorité : 09.11.83 FR 8318077

(43) Date de publication de la demande :
19.06.85 Bulletin 85/25

(45) Mention de la délivrance du brevet :
03.12.86 Bulletin 86/49

(84) Etats contractants désignés :
AT BE CH DE GB IT LI LU NL SE

(56) Documents cités :
S. PATAI et al.: "The chemistry of amides", 1970,
pages 96,97, Interscience Publishers, Londres, GB;
BERICHTE DER DEUTSCHEN CHEMISCHEN GESELL-
SCHAFT, vol. 1, 1903, pages 1278-1283; L. KNORR et
al.: "Zur Kenntnis des Aethanolamins"
JOURNAL OF THE AMERICAN CHEMICAL SOCIETY,
vol. 73, no. 12, 24 décembre 1951, pages 5557-5559,
Washington D.C., US; A.P. PHILLIPS: "Ethanolamides
of some mono- and dicarboxylic acids"

(73) Titulaire : **RHONE-POULENC SPECIALITES CHIMI-
QUES**
**"Les Miroirs" 18, Avenue d'Alsace**
**F-92400 Courbevoie (FR)**

(72) Inventeur : **Crochemore, Michel**
**Domaine de Gilbertin 3, rue des Lilas**
**F-69630 Chaponost (FR)**

(74) Mandataire : **Rioufrays, Roger et al**
**RHONE-POULENC INTERSERVICES Service Brevets·
Chimie Centre de Recherches de Saint-Fons B.P. 62**
**F-69192 Saint-Fons Cédex (FR)**

## Description

La présente invention a pour objet un procédé de préparation de la N,N'-bis (hydroxy-2 éthyl)oxamide par réaction de l'éthanolamine avec un oxalate d'alkyle.

La N,N'-bis(hydroxy-2 éthyl)oxamide est un produit chimique utilisé comme intermédiaire pour la fabrication d'explosifs puissants (cf. M. G. DESSEIGNE Revue des Poudres *30* page 101 [1948] et R. S. STUART et al Can. J. Res. *26* page 402 [1948]). La N,N'-bis(hydroxy-2 éthyl)oxamide est préparée par condensation de l'éthanolamine avec les oxalates de dialkyle. Depuis la publication de L. KNORR et P. ROSSLER, Ber. *36* pages 1278-1279 (1903) cette condensation a été conduite en milieu anhydre, soit en absence de solvant ou dans un excès d'éthanolamine, soit dans un alcool anhydre, cf. A. P. PHILIPPS et al, J. Am. Chem. Soc. *73* page 5557 (1951) ; G. F. D'ALELIO et al, J. Am. Chem. Soc. *59* page 111 (1937) ; R. S. STUART et al loc. cit. ; M. G. DESSEIGNE loc. cit. Ces derniers auteurs insistent particulièrement sur la nécessité de recourir à des solvants anhydres, en particulier à des alcools tels que l'éthanol et le butanol. Les meilleurs rendements en N,N'-bis(hydroxy-2 éthyl)oxamide sont obtenus lorsqu'on utilise les alcools comme solvants. Dans ce cas les rendements par rapport à l'oxalate ou l'éthanolamine dépassent 90 % et peuvent être quantitatifs. Malgré cela ces procédés souffrent de deux inconvénients majeurs qui les rendent inutilisables industriellement. Le premier de ces inconvénients tient à la productivité de la réaction qui se révèle particulièrement faible et de l'ordre de 110 kg/m³ de milieu réactionnel dans le meilleur des cas. Par ailleurs il a été constaté que lorsqu'on utilise un alcool comme solvant la N,N'-bis(hydroxy-2 éthyl)oxamide précipite au fur et à mesure de sa formation sous forme de fines particules gonflées d'alcool ce qui conduit à la formation d'une crème non fluide qui adhère aux parois du réacteur. Cette crème s'avère difficile à extraire de l'appareil de réaction et à filtrer. La préparation à l'échelle industrielle de la N,N'-bis(hydroxy-2 éthyl)oxamide impliquait donc le recours à un milieu réactionnel exempt des inconvénients précités et conduisant néanmoins à l'obtention de bons rendements. La présente invention se propose précisément de résoudre ce problème.

Plus particulièrement la présente invention a pour objet un procédé de préparation de la N,N'-bis(hydroxy-2 éthyl)oxamide par réaction d'un oxalate d'alkyle avec l'éthanolamine caractérisé en ce que l'on opère dans l'eau comme milieu réactionnel.

On a constaté qu'en conduisant la réaction dans l'eau la concentration en réactifs du milieu peut être notablement plus élevée que dans un alcool ce qui se traduit par une amélioration substantielle de la productivité de la réaction qui peut être portée à 400 kg/m³ de masse réactionnelle. Par ailleurs, la N,N'-bis(hydroxy-2 éthyl)oxamide cristallise au fur et à mesure de sa formation sous forme de cristaux dont la séparation par filtration est instantanée.

L'obtention de bons rendements dans ces conditions apparaît inattendue au regard de l'art antérieur qui préconisait l'emploi de solvants anhydres.

Le choix des oxalates d'alkyles de départ n'est pas critique et l'on peut faire appel à tout oxalate d'alcools aliphatiques. Dans la pratique il est préférable, d'un point de vue industriel, de faire appel aux oxalates d'alcools aliphatiques inférieurs tels que les oxalates de méthyle, d'éthyle, de n-propyle, d'isopropyle, de n-butyle.

La quantité d'éthanolamine exprimée en moles par mole d'oxalate est voisine de la stœchiométrie de la réaction soit 2 moles par mole d'oxalate. Naturellement on ne sortirait pas du cadre de la présente invention en utilisant un défaut ou un excès d'éthanolamine, cependant cela ne se traduirait pas par des avantages particuliers.

La température à laquelle la réaction est conduite peut varier dans de larges limites. Ainsi elle peut aller de 0 à 100 °C. Cependant on obtient d'excellents résultats en opérant à une température de 15 à 50 °C. En général il est suffisant que la température soit de l'ordre de 20 à 40 °C.

La concentration des réactifs dans l'eau n'est pas critique toutefois on n'a pas intérêt à opérer en milieu trop dilué si l'on veut conserver une bonne productivité à la réaction. Dans la pratique il est préférable à cet égard que la concentration en éthanolamine dans l'eau soit au moins égale à 2,5 moles/litre et de préférence au moins égale à 5 moles/litre. La concentration maximale dépend de l'agitabilité de la masse réactionnelle.

D'un point de vue pratique il convient d'ajouter progressivement l'oxalate à la solution aqueuse d'éthanolamine au fur et à mesure du déroulement de la réaction. En opérant de cette façon on évite l'hydrolyse de l'oxalate. La durée de l'addition dépend des conditions de la réaction : température, dilution et efficacité de l'agitation.

La N,N'-bis(hydroxy-2 éthyl)oxamide précipite sous forme de fins cristaux au fur et à mesure de sa formation. On obtient de cette façon un produit de très grande pureté dont la filtration est instantanée. Le filtrat consistant en une solution aqueuse de N,N'-bis(hydroxy-2 éthyl)oxamide, d'alcool résultant de la condensation et éventuellement d'alcool de lavage du précipité est distillé pour éliminer l'alcool. La solution aqueuse de N,N'-bis(hydroxy-2 éthyl)oxamide ainsi récupérée est de préférence utilisée pour une nouvelle opération de condensation.

Les exemples suivants illustrent l'invention et montrent comment elle peut être mise en pratique.

## Exemple 1

Dans un ballon en verre de 1 litre, muni d'un thermomètre, d'un agitateur en forme de demi-lune, d'une ampoule de coulée et refroidi par un bain d'eau froide, on charge 200 ml d'eau distillée et 183 g (3 moles) d'éthanolamine. On agite et obtient une solution aqueuse d'éthanolamine dont la température atteint 35 °C. On refroidit cette solution à 25 °C puis ajoute sous agitation en 1 heure 219 g (1,5 mole) d'oxalate d'éthyle en maintenant la température à 25 °C. La cristallisation de la N,N'-bis(hydroxy-2 éthyl)oxamide commence lorsque le tiers de l'oxalate a été ajouté. On maintient la masse réactionnelle à 25 °C sous agitation pendant 15 minutes après la fin de l'addition. On refroidit le contenu du ballon à 3 °C et filtre sur verre fritté. La filtration est immédiate. Le gâteau est lavé par 150 cm³ d'alcool absolu puis on le sèche sous vide à 50 °C jusqu'à poids constant.

On recueille de cette façon 220 g d'un produit présentant un point de fusion (banc de Kofler) de 169 °C et dans lequel on dose 100 % de N,N'-bis(hydroxy-2 éthyl)oxamide par chromatographie liquide sous pression. La nature et la pureté du produit sont confirmées par spectrographie IR, RMN et de masse.

On récupère en outre 445 cm³ de jus-mères dans lesquels on dose 24 g de N,N'-bis(hydroxy-2 éthyl)oxamide en solution. Il s'est donc formé au total 244 g d'oxamide ce qui correspond à un rendement de 92 % par rapport à l'éthanolamine et à l'oxalate mis en œuvre. Le rendement en produit pur isolé s'élève à 83 %.

## Exemple 2

On réalise un premier essai en suivant le mode opératoire décrit à l'exemple 1. Après filtration et lavage du gâteau on réunit le filtrat et l'alcool de lavage puis distille l'alcool de lavage et l'alcool de réaction à pression atmosphérique à l'aide d'une colonne Vigreux de 220 mm de hauteur et 24 mm de diamètre. On recueille 3 012 cm³ d'alcool à 95 % sous forme de distillat et 145 cm³ d'une solution aqueuse de N,N'-bis(hydroxy-2 éthyl)oxamide que l'on charge dans le ballon réactionnel. On répète la condensation en ajoutant à nouveau 183 g d'éthanolamine puis 219 g d'oxalate d'éthyle puis on traite la masse réactionnelle comme ci-avant en lavant le gâteau à l'aide de l'alcool récupéré. L'opération est reproduite 5 fois. On a chargé au total 1 314 g d'oxalate d'éthyle (9 moles) et 1098 g (18 moles) d'éthanolamine. On a recueilli au total 1 451 g de N,N'-bis(hydroxy-2 éthyl)oxamide à 100 % soit un rendement moyen de 91,6 %.

## Exemples 3 à 6

On a reproduit l'exemple 1 en faisant varier les conditions de la réaction selon les indications du tableau suivant dans lequel sont également consignés les résultats obtenus.

| EXEMPLES | REACTIFS | | EAU en ml | T°C | DUREE | | OXAMIDE | | RR % | |
|---|---|---|---|---|---|---|---|---|---|---|
| | Amine | Oxalate | | | Coulée | Finition | Isolé | Dissous | Isolé | Total |
| 3 | 122 g | 146 g | 400 | 25° | 2 h | 2 h | 112 | 21 | 63,6 | 75,5 |
| 4 | 122 g | 146 g | 200 | 25° | 2 h | 2 h | 140 | 12 | 79,5 | 86,5 |
| 5 | 183 g | 219 g | 200 | 25° | 2 h | 2 h | 219 | 24 | 83 | 92 |
| 6 | 183 g | 219 g | 200 | 80° | 2 h | 1 h | 194 | 13,5 | 73,5 | 80,5 |

## Revendications

1. Procédé de préparation de la N,N'-bis(hydroxy-2 éthyl)oxamide par réaction d'un oxalate d'alkyle avec l'éthanolamine caractérisé en ce que l'on opère dans l'eau comme milieu réactionnel.

2. Procédé selon la revendication 1, caractérisé en ce que l'oxalate est ajouté progressivement à une solution aqueuse d'éthanolamine.

3. Procédé selon l'une quelconque des revendications 1 à 2, caractérisé en ce que la température de la réaction est comprise dans un intervalle de 0 à 100 °C.

4. Procédé selon l'une quelconque des revendications 1 à 3, caractérisé en ce que la concentration de la solution aqueuse d'éthanolamine est d'au moins 2,5 moles par litre d'eau.

## Claims

1. Process for the preparation of N,N'-bis (2-hydroxyethyl)oxamide by reaction of an alkyl oxalate with ethanolamine, characterized in that it is carried out in water as the reaction medium.

2. Process according to Claim 1, characterized in that the oxalate is gradually added to an aqueous ethanolamine solution.

3. Process according to either of Claims 1 to 2, characterized in that the reaction temperature is in a range from 0 to 100 °C.

4. Process according to any one of Claims 1 to 3, characterized in that the concentration of the aqueous ethanolamine solution is at least 2.5 moles per litre of water.

## Patentansprüche

1. Verfahren zur Herstellung von N,N'-Bis(hydroxy-2-ethyl)oxamid durch Reaktion eines Alkyloxalats mit Ethanolamin, dadurch gekennzeichnet, daß man in Wasser als Reaktionsmedium arbeitet.

2. Verfahren nach Anspruch 1, dadurch gekennzeichnet, daß das Oxalat fortschreitend zu einer wässrigen Lösung von Ethanolamin zugesetzt wird.

3. Verfahren nach Anspruch 1 oder 2, dadurch gekennzeichnet, daß die Reaktionstemperatur im Bereich von 0 bis 100 °C liegt.

4. Verfahren nach einem der Ansprüche 1 bis 3, dadurch gekennzeichnet, daß die Konzentration der wässrigen Ethanolaminlösung mindestens 2,5 Mol pro Liter Wasser beträgt.